# EUROPEAN PATENT APPLICATION

(11) **EP 4 821 352 A1**
(43) Date of publication of application: **14.10.2026**
(21) Application number: 25169239.8
(22) Date of filing: 08.04.2025
(51) Int. Cl.: A01K 67/0278, A01K 67/0275, C07K 16/00, C12N 15/85

(54) **TRANSGENIC RODENT EXPRESSING A REDUCED COMPLEMENT OF IMMUNOGLOBULIN CONSTANT DOMAINS**

(71) Applicant: AnsoThera uG, 91088 Bubenreuth (DE)
(72) Inventor: Killeen, Nigel, Hillsborough, 94010 (US); Schulz, Sebastian R., 91058 Erlangen (DE); Jäck, Hans-Martin, 91088 Bubenreuth (DE)
(74) Representative: Grünecker Patent- und Rechtsanwälte PartG mbB

(57) **Abstract**

This invention concerns a transgenic mouse platform for generating therapeutic antibodies that would be appropriate for the treatment of diseases in humans or in animals that are of veterinary significance. As examples, bovine, feline, and canine variable domains may be present in the antibody repertoires. In all cases, the antibodies in the repertoires are chimeric carrying constant domains that have a different species origin to the variable domains. The invention is distinguished by usage of a restricted complement of immunoglobulin constant domains permitting robust B lymphocyte development, antigen-specific responses, and efficient somatic hypermutation when used in a mouse-expressed antibody repertoire.

## Description

This invention concerns a transgenic rodent, preferably mouse, platform for generating therapeutic antigen-binding molecules that would be appropriate for the treatment of diseases in humans or animals, including specifically, animals that are of veterinary significance. The transgenic rodents, preferably mice, carry large-scale modifications to the genomic loci that encode their immunoglobulins. By virtue of these modifications, the rodents, preferably mice, gain the capacity to produce diverse repertoires of chimeric antigen-binding molecules.

Three key features are preferred for the chimeric molecules. Firstly, the sequences encoding their antigen-binding domains are not native to the cognate mouse immunoglobulin loci. Secondly, the genomic regions encoding the antigen-binding domains derive from germline gene segments that have a synthetic (i.e., designed) origin. Thirdly, the chimeric molecules comprise immunoglobulin constant domains from a species other than mouse.

As examples, antigen-binding domains in the chimeric repertoires may comprise bovine, feline, or canine immunoglobulin variable domains, human T cell receptor variable domains, or domains that are not normally diversified by the RAG-1/2 recombinase.

The invention may also be further distinguished by usage of a restricted complement (i.e., repertoire) of immunoglobulin constant domains as compared to non-transgenic rodents, preferably not transgenic mice. The invention offers efficiency advantages for antibody discovery by reducing the number of constant domains available and by the incorporation of a temporal marking capability.

The invention is permissive of robust B lymphocyte development, antigen-specific humoral responses, and efficient somatic hypermutation of the chimeric antigen-binding molecules. The rodents, preferably mice, can be used in a broad range of immunization protocols to yield antigen-binding domains that could be the basis of novel biological therapeutics.

### BACKGROUND OF THE INVENTION

Antibodies are proteins that protect vertebrate species against infectious agents. They accomplish this in a variety of ways, all of which depend on specific binding between an antibody and a component of the infecting entity. Vertebrate immune systems are possessed of powerful mechanisms for diversifying their antibodies such that a given member of a species may be protected against a multitude of pathogens by hundreds of millions of different antibodies.

With some species-specific exceptions, antibodies are comprised of two kinds of protein chains, one of them a heavy chain of about 50kDa and the other a light chain of about 25kDa. In immunoglobulin gamma (IgG)-type antibodies, two heavy chains are found in a dimer, and each of these is itself hetero-dimerized with a light chain at its amino-terminal end. This composition allows IgG antibodies to assume a Y-shape, with the carboxy-terminal half of each heavy chain found dimerized in the stem of the Y and the two heterodimeric parts (each comprised of both heavy and light chains) constituting its arms.

Sequences that account for the capacity of antibodies to bind to different infectious targets are localized to single domains (each of about 110 amino acids) at the amino-terminal ends of both kinds of chains. These are called variable domains. A novel composite protein surface is created when heavy and light chain variable domains heterodimerize, and it is here that target engagement occurs. The targets bound by antibodies are called antigens.

Genomic rearrangement is a primary means for diversifying the variable domains of antibodies. This occurs in immature lymphocytes throughout life, and results in a large multiplicity of clones of mature cells (B lymphocytes and plasma cells) differing in the antibodies they produce.

The coding sequence for individual variable domains derive from rearrangement events that join two or three pieces of DNA together. In their germline configuration these 'gene segments' may be separated from one another by kilobases to megabases of intervening DNA. Large numbers of gene segments are found in immunoglobulin loci. For example, the reference sequence for the human heavy chain locus contains 127 variable (V), 27 diversity (D), and 9 joining (J) gene segments (see Watson et al. (2013) Am J Hum Genet. 92(4):530-546; Rodriguez et al. (2023) Nature Communications 14:4419). The genomic loci encoding immunoglobulin light chains are similarly comprised of numerous V and J gene segments.

Genomic rearrangement at immunoglobulin loci depends on regulated expression of the RAG-1/2 recombinase in immature cells. This enzyme creates V-D-J or V-J gene segment fusions in heavy or light chain alleles, respectively.

Antibody protein sequence diversity in many vertebrate species derives from the following:
1. paralogous gene segment sequence differences;
2. random usage of V, D and J gene segments in different clones of antibody-expressing cells;
3. imprecision in the joining of V, D and J gene segments;
4. post-rearrangement mutation or replacement of variable domain exons.

Some vertebrate species also rely on gene conversion as a primary or supplementary mechanism of diversifying antibody variable domains.

As mentioned above, variable domain sequence diversity creates complex repertoires of antibody-expressing cells, a commensurate capacity to engage a similarly complex population of antigens, and a dependent ability to provide protection against numerous pathogens.

The antibody heavy chain locus is now known to be one of the most polymorphic regions of the human genome. Sequence diversity at this locus (and probably also at light chain loci) can underpin individual-specific differences in antibody repertoires, which in turn can impact pathogen responsiveness and autoimmune susceptibilities. Such polymorphism also contributes to why humans differ from other species in the antibody repertoires they express.

Antibody heavy chains are comprised of four or five discrete polypeptide domains, each of which is said to adopt an immunoglobulin folding pattern. Light chains, by contrast, are comprised of only two such domains. In each case, the amino-terminal (variable) domain is the one that is diversified by genomic rearrangement. The other domains are individually, or in aggregate, referred to as 'constant' domains.

In addition to V(D)J rearrangement, the immunoglobulin heavy chain locus is modified by a process known as class switch recombination. Whereas V(D)J rearrangement is responsible for diversifying the exon encoding the heavy chain variable domain, class switch recombination causes a change in the part of the locus that encodes the constant domains. In its germline state, the locus is configured to produce both IgM and IgD heavy chains, but after switch recombination, it instead produces IgG, IgA or IgE heavy chains. These different constant domains confer distinct immunological properties on antibodies such as the capacity to cross the placenta or to bind avidly to Fc receptors on disparate populations of immune cells.

While the primary natural function of antibodies is to provide protection against infections, they have been exploited increasingly over the past fifty years as tools for a broad range of research, diagnostic, and clinical purposes. This versatility derives from the fact that antibodies can be created to target a broad range of molecular forms from small chemical compounds to complex protein surfaces. It also derives from the relative ease with which new antibodies can now be generated using a plurality of powerful discovery technologies and platforms.

In the clinic, antibodies have become a mainstay of modern medicine. They are used to treat a range of human conditions that includes cancer, organ transplant rejection, inflammatory and autoimmune disorders, infections, osteoporosis, eye conditions, migraines, high cholesterol and nervous system disorders. There are now more than 120 therapeutic antibodies approved for use in the United States and a similar number in the European Union. In both locales, there are also many new antibodies being reviewed for approval. The global market for therapeutic antibodies is expected to generate about a quarter trillion US dollars in revenue in 2024, rising to about a half trillion by 2029.

Therapeutic antibodies can be a target of patient immune responses that limit their effectiveness. Such immune responses are highly likely if the antibodies are comprised of immunoglobulin chains from other species because of the polymorphism mentioned above. Therapeutic antibodies for use in humans are therefore comprised of human immunoglobulin chains, and/or chains that are mostly of human origin. This requirement creates a need for methodologies that allow for the routine discovery and isolation of fully human antibody variable domains. One such methodology that has proven especially effective in this regard involves transgenic mice that express human antibody variable domain repertoires.

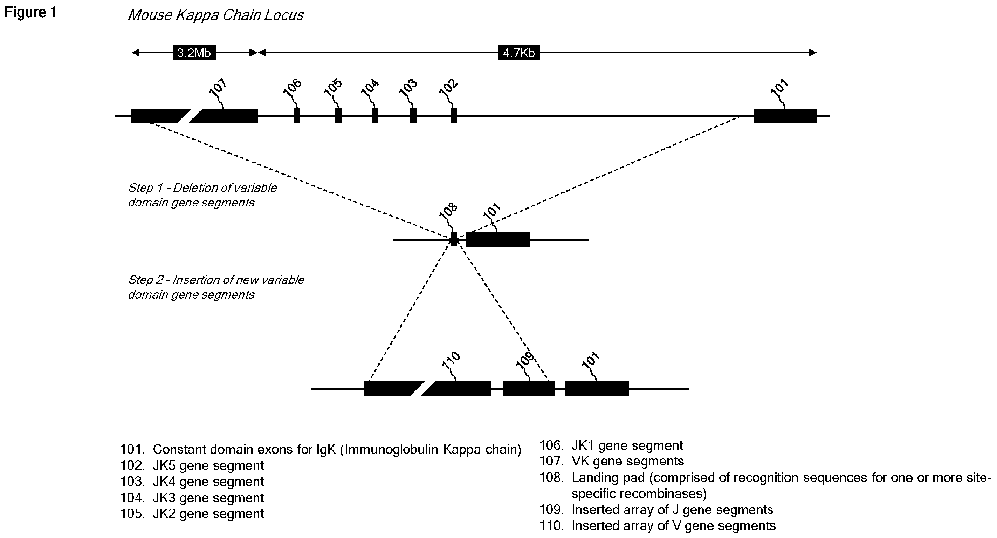

Among various strategies for creating transgenic mice expressing human antibody variable domain repertoires, the most effective has involved a two-step approach (Figure 1). In the first step, the endogenous gene segments that are used to express antibody variable domains are deleted from the genome (or rendered inaccessible). This step is then followed by a replacement procedure that inserts human antibody variable domain gene segment DNA into the location that previously harbored the mouse gene segments. The result of this process is a hybrid locus structured such that it can be rearranged by the RAG-1/2 recombinase to generate human variable domain diversity that may be expressed together with host (*i.e.,* mouse) constant domains as part of a chimeric antibody repertoire. In practice, mice generated by this kind of procedure are possessed of broad variable domain repertoire diversity equivalent to that found in humans. They have proven highly effective as platforms for the isolation of therapeutic antibodies.

Mice - partially or entirely - of the sort just described have been generated using a variety of approaches and technologies (see for example, Lonberg et al. (1994) Nature 368:856-9; Green et al. (1994) Nature Genetics 7:13-21; Nicholson et al. (1999) J. Immunol. 163:6898-906; Murphy et al. (2014) Proc Natl Acad Sci USA 111:5153-8; Lee et al. (2014) Nature Biotechnology 32: 356-363; Brüggemann et al. (2015) Arch. Immunol. Ther. Exp. 63:101-108; Xu et al. (2021) Nature 595: 278-282).

The genetic loci encoding immunoglobulin chains have been sequenced and annotated in multiple species (see IMGT.org; see also Das et al. (2008) Immunogenetics 60:47-55). These loci can extend to several megabases in size, and in some cases, can contain more than a hundred functional gene segments. It is a feasible, but technically challenging endeavor to transfer large loci of this sort into the mouse genome to create transgenic animals expressing antibody chains from other species. The process can, however, be simplified by creating designed synthetic arrays of gene segments and using these much smaller moieties in place of their natural counterparts (see US patents 10,881,084, 10,662,256, 10,793,829, 12,049,516).

In principle, synthetic arrays can be created that contain gene segments from an immunoglobulin locus of any species. If the arrays are properly constructed, the gene segments can undergo V(D)J rearrangement in immature B cells to create variable domain exons from only the foreign gene segments. Insertion of such arrays into the appropriate locations of mouse immunoglobulin loci will create alleles capable of being rearranged to express chimeric antibody chains comprised of mouse constant domains linked to foreign variable domains. Depending on the complexity of the synthetic gene segment array, an allele of this sort can be possessed of the capacity to approximate the full extent of variable domain repertoire diversity found in the source species.

The technology just summarized was developed to allow for expression of human antibody variable domain repertoires in transgenic mice. Such mice have subsequently been used for the isolation of therapeutic antibodies for treating an expanding range of human diseases.

Antibodies are now being developed as therapeutics for the treatment of diseases in animals of veterinary significance, such as cats, dogs, and cattle, among others. To date, these include USDA/FDA-approved antibodies for the treatment of allergic dermatitis and atopic dermatitis in dogs, parvovirus in dogs, osteoarthritis in dogs, and pain associated with osteoarthritis in cats.

Just as with human therapeutic antibodies, there is a need for discovery platforms that will simplify the isolation of new antibodies for use in such animals. These platforms must be tailored for the particular species of interest for the same reasons that have driven the development of the human antibody discovery platforms mentioned above; that is, so that the antibodies isolated and the animals to be treated share a common species origin, thereby greatly reducing the likelihood of a problematic anti-drug immune response during therapy.

Discovery platforms are being developed for the isolation of therapeutic antibodies for an expanding range of animal species. For example, US10,662,256B2, US10,793,829 B2, US2024/0215556, US2024/0206440 A1, and WO2023247779A1 disclose transgenic mice expressing repertoires of chimeric antibodies comprised of mouse constant domains operationally linked to variable domains of bovine, canine, feline and equine origin.

Antibody discovery platforms of the sort summarized above involve engineered immunoglobulin alleles that typically retain the native exons encoding mouse constant domains. This reflects early experience with immunoglobulin transgenes in which suboptimal performance - for antibody discovery purposes - was attributed to their usage of human heavy chain constant domains.

Mouse heavy chain constant domains allow for good B cell antigen receptor signaling and normal functionality with mouse Fc receptors. Both contributions are necessary for efficient B cell development and somatic hypermutation, and in turn, these are key properties of transgenic mouse antibody discovery platforms that are currently established in the art.

The present transgenic mouse antibody discovery platforms require further improvements, e.g. in terms of simplifying antibody discovery derivable from these platforms.

The mouse heavy chain locus has the capacity to produce antibodies with eight different kinds of constant domains (namely, IgM, IgD, IgG1, IgG2a or IgG2c, IgG2b, IgG3, IgA, and IgE). While this complexity is important for humoral immunity, it is typically not of value for routine antibody discovery. Instead, the complexity can complicate the discovery of some antibody variable domains, particularly those that might predominate in non-IgG antibodies and thus might be difficult to isolate using certain methodologies.

### SUMMARY OF THE INVENTION

The present inventors surprisingly found that the performance and versatility of a transgenic mouse antibody discovery platform can be improved by simplifying the part of the heavy chain locus that contains constant domain exons.

On the one hand, such simplification has the potential to facilitate the isolation of antigen-specific B cells - and the antibodies they express - using technology that depends on constant region discrimination. On the other hand, the simplification can provide the basis for the installation of additional genetic modifications designed to enhance access to the antigen-specific repertoire and thereby to minimize resources required to isolate antibodies with desired characteristics. The performance of the transgenic mouse antibody discovery platform is thereby not diminished and preferably improved as compared to the prior art. The concept of the present invention, herein described for mouse platforms is applicable to transgenic rodents in general.

The invention described here, thus, provides an optimized transgenic rodent platform for the discovery of antibodies. The present invention, compared to the prior art, provides an improved transgenic rodent that is genetically engineered to provide a repertoire of chimeric antibodies with a restricted spectrum of immunoglobulin constant domains.

A "restricted spectrum of immunoglobulin constant domains" or "reduced complement of immunoglobulin constant domains" means that the rodent is genetically modified to express antibodies comprising fewer different immunoglobulin constant domains than a natural, original or an unmodified animal of the same species, preferably a laboratory animal of the same species. Preferably a rodent, preferably mouse, which is a "natural", "original" or an "unmodified" animal of the same species, exhibits the natural repertoire of immunoglobulin domains, e.g. heavy chain constant domains.

Likewise, the present invention in a different aspect contemplates transgenic rodents, preferably mice, exhibiting a spectrum of immunoglobulin constant domains that is altered compared to native rodents, preferably native mice, in that one or more of the exons encoding native constant domains are deleted. Replacement by non-native exons is possible in line with the main aspects of the present invention.

A "restricted spectrum of immunoglobulin constant domains" or "reduced complement of immunoglobulin constant domains" is, thus, preferably relative to the natural complement of immunoglobulin heavy chain constant domains found in common strains of laboratory rodents.

In one aspect of the invention, a transgenic rodent, preferably a mouse, is provided, which is genetically engineered to be capable of expressing a functional repertoire of chimeric antibodies with a restricted complement (i.e. repertoire) of immunoglobulin heavy chain constant domains; preferably compared to the natural repertoire of immunoglobulin heavy chain constant domains found in non-transgenic rodents, preferably, if the rodent is a mouse, in common strains of laboratory mice (such as the C57BL/6 or BALB/c strains).

In a further aspect of the invention, a method for generating the transgenic rodent of the present invention is provided, said method comprising,
a) deleting variable domain gene segments from an immunoglobulin locus in the rodent genome, and
b) reinserting arrays of variable domain gene segments into the modified immunoglobulin locus,
wherein steps a) and b) are preferably conducted in embryonic stem (ES), cells of the rodent, or in fertilized rodent oocytes.

The present invention features multiple innovations intended to improve the performance of the platform over alternative versions. Specifically, the innovations may be designed to facilitate the identification and isolation of antigen-specific B cells. In turn, these may be used to discover the coding sequences of the relevant antigen-binding moieties expressed.

In a further aspect of the invention, a method for obtaining antibodies or antigen-binding domains or for discovering the coding sequences of antibodies or antigen-binding domains is provided comprising the immunization of the transgenic rodent, preferably the transgenic mouse, of the present invention.

The antibodies produced by the transgenic rodent of the present invention or the B cells of the present invention are preferably chimeric, e.g. they partially comprise sequences that are not native to the mouse immunoglobulin locus but are instead derived from humans or other species such as those of veterinary significance like bovine, feline, or canine species.

The core features of the platform, thus, allow for the isolation of antibodies for use in humans or animals of veterinary significance. They also permit the isolation of non-antibody antigen-binding moieties, such as T cell antigen receptor variable domains, and synthetic antigen-binding domains (exemplified by adnectins, anticalins, DARPins and knottins).

The invention represents an advance over contemporary transgenic mouse platforms because of the novel performance-focused innovations it comprises, particularly those related to a restricted constant domain complement.

The species to be used as the source for variable domain gene segments that are inserted into the invention's immunoglobulin loci is generally left open; it is specified only that these are "human" or "of veterinary significance" or "of veterinary interest". In preferred embodiments, antibody variable domains of veterinary significance or of veterinary interest are derived from cattle, dogs, horse, cats, and birds. The birds may be chickens, pheasants, turkeys, ducks, Guinea fowl, Houbara bustard, or other avian species.

In the transgenic rodent, preferably mouse, of the present invention one or more of their native constant domain-encoding exons are deleted and therewith the capability of producing the one or more of these native constant domains is removed. Preferably, the transgenic rodent, preferably mouse, further includes a constant domain-encoding part of the locus that is reconfigured so that it expresses one or more constant domains not present in the native rodent, more preferably these constant domains include one or more of IgM-, IgD-, IgG2b-, IgG1-, and IgA- constant domains. Even more preferably, in the transgenic rodent, preferably mouse, of the present invention, the capacity to produce rodent, preferably mouse, antibody variable domains from immunoglobulin loci has been eliminated. Further, even more preferably, in the transgenic rodent, preferably mouse, of the present invention, synthetic gene segments are inserted into the immunoglobulin loci endowing them with the capacity to produce chimeric immunoglobulin chains that partially comprise variable domains of "human" or "of veterinary significance" or "of veterinary interest", or alternatively antigen-binding domains that are not derived from antibody-encoding loci.

"Native" or "original" refers to a respective rodent, preferably respective mouse, that is not genetically engineered.

The invention in a preferred embodiment, develops further a two-step concept of genome engineering involving an elimination of the capacity to produce rodent, preferably mouse, antibody variable domains from immunoglobulin loci, together with a reconstitution of variable domain producing capacity by inserting back into the loci designed synthetic pieces of DNA comprising arrays of variable domain gene segments.

In a preferred embodiment, the rodent, preferably mouse, locus that normally encodes immunoglobulin heavy chains is modified by two deletion events. The first of these removes the variable domain gene segments (V_{H} gene segments) while the second removes the D_{H} and J_{H} gene segments and all the constant domain-encoding exons, leaving in place one or both *Adam6* genes. These deletion steps are followed by a replacement step in which the constant domain-encoding part of the locus is reconfigured so that it expresses constant domains that can include one or more of IgM, IgD, IgG2b, IgG1, and IgA, preferably one or more of rat IgM, IgD, IgG2b, IgG1, and IgA. Finally, a second replacement step (or more than one such step, if necessary) inserts synthetic arrays of V_{H}, D_{H}, and J_{H} gene segments into the engineered locus. The inserted gene segments have coding sequences taken from humans or a species of veterinary significance. The resultant locus can be recombined by the RAG-1/2 recombinase to allow for expression of chimeric immunoglobulin heavy chains comprising variable domains from the selected species of veterinary significance and rat constant domains.

In a related fashion to that just described, the transgenic, preferably mouse, loci encoding the Kappa and lambda immunoglobulin light chains can be modified by an engineered deletion event that removes all the V_{L} and J_{L} gene segments they contain. The deletions similarly delete the constant domain-encoding exons from the loci. Synthetic arrays of gene segments can then be inserted into the two loci, their constituent gene segments comprising coding sequences from humans or a species of veterinary significance. The inserted arrays also contain exons encoding rat light chain constant domains (rat C_{Kappa} for the Kappa locus, and rat C_{Lambda} for the Lambda locus). In a more preferred embodiment of the invention the gene segments in the synthetic arrays used in all three immunoglobulin loci (encoding the heavy, lambda, and kappa chains) have a common species origin for their coding sequences, that species being human, or of veterinary significance.

The invention features modifications to the constant domain-encoding part of the mouse immunoglobulin heavy chain locus. These modifications constitute a simplification of the locus in that they reduce the number of constant domains it can express (from eight, which is the normal number, to three or four, or at most five). The modifications further include a change in the species origin of the constant domains - instead of mouse constant domains, the engineered locus will express chimeric antibody molecules comprising rat constant domains or other non-mouse constant domains that allow for efficient BCR signaling while also interacting productively with mouse Fc receptors. Finally, the modifications include the placement of recognition sequences for site-specific recombinases (e.g., the Dre recombinase) in such a way that expression of the cognate recombinase would result in a serologically evident change of constant domain identity (such as, for example, from IgG2b to IgG1). A conditional change of this sort can be used to discriminate antigen-specific B cells with distinctive desirable experiences from other B cells.

The design of the engineered immunoglobulin loci present in the invention is permissive of efficient V(D)J rearrangement by the RAG-1/2 recombinase in immature B cells. This creates a diverse repertoire of antigen receptors, which in turn is sufficient for robust B cell development. The engineered loci are accessible for normal somatic hypermutation during germinal center reactions, and thus the antibodies expressed from them can undergo a normal form of affinity maturation. All these features endow the mice with the capacity to make strong antibody responses to a broad range of antigens using the same kinds of immunization protocols as would be used with other strains of laboratory mice. Meanwhile, the engineering innovations included in the mice - especially those in the constant-domain-encoding part of the heavy chain locus - are designed to facilitate the identification and isolation of antigen-specific B cells thereby increasing the likelihood of discovering therapeutic candidates with desirable properties.

Antibody discovery campaigns typically involve repeated immunizations of mice with a single antigen, or with more than one closely related variant of a single antigen (with different variants being used in successive immunizations). Depending on the goals of the campaign and the nature of the antigen being used, it can be useful to have a means for discriminating antigen-specific B cells (and the antibodies they express) that derive from primary versus later (i.e., secondary, tertiary, etc.) germinal centers or vice versa. This kind of discrimination has the potential to focus a discovery campaign on antibodies specific for certain kinds of epitopes and thereby can reduce the effort required to isolate such antibodies. The engineering of the constant domain-encoding part of the heavy chain locus that is part of this invention allows for such discrimination.

Secondary immunizations (i.e., booster immunizations) normally result in antibody responses that are stronger than those produced by primary immunizations. Strength in this context is a consequence of better antibody titers (i.e., the concentration of antibodies in serum is increased, in some cases by one or two orders of magnitude) but also improved antibody affinity (due to affinity maturation that occurred in the germinal centers of the primary immune response). It is now understood that pre-existing antibodies and an abundance of memory T cells help to focus secondary (and later) antibody responses in a fashion that provides protection against antigenic variation in pathogens. While this kind of focusing can be useful for many simple antibody discovery campaigns, it may complicate campaigns designed to isolate antibodies that have undergone successive rounds of affinity maturation during sequential immunizations. As an example, broadly neutralizing antibodies specific for viral infections such as HIV or influenza are a class of antibodies that is associated with extensive somatic hypermutation that may require extended involvement in germinal centers in this fashion.

The features present in this invention are designed to permit the discrimination of B cells (and the antibodies they express) that have participated in the germinal center reactions elicited by one of several possible immunizations in an antibody discovery campaign. As such, they provide a means for enriching for B cells that have had certain kinds of somatic hypermutation experiences and thus might be enriched for the expression of antibodies with distinct antigen-binding properties. Such properties could include atypical binding kinetics, engagement of subdominant epitopes, cross-reactivity (or the lack of it) to related antigens, elimination of binding to secondary targets, acquisition or enhancement of enzymatic properties, improved stability in certain biological milieus, or antigen recognition that is contingent on such milieus.

In the prior art up to date, antibody discovery campaigns do not typically involve manipulations that alter pre-existing antibody levels (i.e., antibodies present prior to secondary or later immunizations). According to the present invention, however, such manipulations can be exploited to increase the formation of antibodies with properties such as those just mentioned (i.e., properties that might be infrequent or absent in the antibodies resulting from standard immunization procedures). The constant domain modifications included in this aspect of the invention provide a means for identifying antigen-specific B cells that might be of most interest in the context of manipulations of this sort. They may also constitute a useful genomic foundation for additional engineering in furtherance of accomplishing the desired manipulations using genetics.

In one embodiment of the present invention, during B cell development, the variable domains in the chimeric proteins are diversified by V(D)J rearrangement of the synthetic gene segment array inserted into the heavy chain locus. After V(D)J rearrangement but prior to class switch recombination, the locus will be a source of both IgM and IgD heavy chains as a consequence of normal mRNA alternative splicing. Preferably, the heavy chain locus will have the capacity to undergo class switch recombination, e.g., due to the presence of a switch region immediately upstream of the IgM constant domain exons, and/or at least one switch region downstream of the IgD transcription termination sequence and upstream of exons encoding an alternative constant domain. In this manner, a class switch from IgM and/or IgD to another class is envisaged.

In a more preferred embodiment, the downstream switch region precedes exons encoding an IgG constant domain, such as rat IgG2b. Optionally, the exons encoding this IgG constant domain are followed by a third switch region preceding exons encoding the rat IgA constant domain.

Another more preferred embodiment of the invention includes exons encoding a second kind of IgG constant domain, such as rat IgG1. This second set of exons is placed downstream of the exons encoding rat IgG2b. In this embodiment, both sets of exons encoding IgG constant domains are preceded by a recognition sequence for a site-specific recombinase (e.g., the *roxP* site that is recognized by the Dre recombinase). These sites are placed so that Dre recombination excises the upstream IgG constant domain exons (for rat IgG2b) leaving only the downstream IgG constant domain exons (for rat IgG1). A locus with these features is capable of effecting a switch from encoding a heavy chain with a rat IgG2b constant domain to one that has a rat IgG1 constant domain, contingent on expression of the Dre recombinase. If the Dre recombinase is only expressed in germinal center B cells, for example, then only those B cells undergoing affinity maturation can make the switch. Furthermore, if the Dre recombinase is engineered to be dependent on tamoxifen for nuclear localization, then recombination only occurs in germinal center B cells when tamoxifen is administered.

In the embodiment just described, the downstream IgG exons (encoding the rat IgG1 constant domain) would not be immediately preceded by a switch region. For IgG1 heavy chains to be expressed, therefore, the following would need to occur: 1. switch recombination excising IgM and IgD exons; 2. Dre recombination excising IgG2b-encoding exons. The first of these would typically occur immediately prior to entry of B cells into germinal centers, while the second would occur in the germinal centers if a germinal center-restricted Dre recombinase allele is used and if the mice are also treated with tamoxifen.

According to these embodiments, an AID (activation-induced cytidine deaminase) -dependent class switch from IgM to IgG or IgA is envisaged. Likewise, a switch from one IgG isotype to another (such as from IgG2b to IgG1) through the action of a site-specific recombinase (such as Dre recombinase) is also envisaged.

In a preferred aspect of the invention, in embodiments directed to class switch, the immunoglobulin heavy chain locus of a mouse is structured such that it produces chimeric antigen-binding proteins comprising rat constant domains and variable domains that have their sequence origin in species that are human or of veterinary significance.

Cells expressing rat IgM, IgD, IgG2b or IgG1 can be readily discriminated from one another using commercially available serological reagents (i.e., affinity purified immune sera, or monoclonal antibodies specific for the individual constant domains). Similarly, such reagents can also be used to discriminate and quantify serum antibodies comprising these heavy chains. Rat constant domains efficiently participate in BCR signaling in mouse cells; they also bind well to mouse Fc receptors. Collectively, these features mean that rat immunoglobulin constant domains are a good substitute for mouse constant domains, especially in the context of chromosome engineering designed to simplify the constant domain-encoding part of the heavy chains locus.

A reduced capacity to diversify the constant domain of immunoglobulin heavy chains is a defining aspect of this invention. In a preferred embodiment, the heavy chain locus is a source of proteins comprising rat IgM, IgD, IgG2b, or IgG1 constant domains. This contrasts with the normal laboratory mouse heavy chain locus, which is a source of proteins comprising twice as many kinds of constant domains. Indeed, the engineered locus can express just two kinds of constant domain after class switch recombination, namely rat IgG2b and rat IgG1, the latter requiring Dre recombination before it is available for use. As mentioned, an alternative embodiment of the invention is also capable of expressing heavy chains comprising IgA constant domains, but even in this case the total diversity of constant domains available for use in the antibody repertoire is reduced relative to the normal situation.

Preferably, the transgenic rodent, preferably mouse, according to the present invention expresses immunoglobulin chains comprising human, bovine, equine, feline, or canine variable domains. These antibodies are preferably useful for the development of therapeutic antibodies for humans, cats, dogs or cattle.

According to the present invention, the transgenic rodent, preferably mouse, expresses antibodies with heavy chains that have constant domains comprising rat coding sequences. Preferably, the number of constant domain isotypes expressed in the mice is reduced relative to the number found in normal mice.

In a further aspect of the present invention, stem cells derived from the transgenic rodent are provided. The genome of the stem cells carries the three engineered immunoglobulin loci (encoding antibody heavy, kappa, and lambda chains) in their germline state (i.e., free of V(D)J rearrangements mediated by the RAG-1/2 recombinase, free of class switch recombinations, and free of recombination by the Dre recombinase). The stem cells could be embryonic, endothelial, hematopoietic, or of other origin. Preferably, the stem cells have the capacity to differentiate into B lymphoid progenitor cells and from these into B cells and plasma cells. The invention thus also provides for these differentiated cell types.

In a further aspect of the present invention, a method for generating the transgenic rodent of the present invention is provided, said method comprising,
a) deleting pieces of DNA from the rodent genome, these pieces containing:
   (i) gene segments (including V, D, and J gene segments) that are normally rearranged in B lymphocytes to allow for expression of antibody variable domains;
   (ii) exons that encode antibody constant domains;
b) reinserting a new pieces of DNA into the modified immunoglobulin loci, these pieces containing:
   (i) gene segments (including V, D, and J gene segments) that are normally rearranged in B lymphocytes to allow for expression of antibody variable domains in humans or in species of veterinary interest;
   (ii) exons that encode rat antibody constant domains;
wherein steps a) and b) are conducted in embryonic stem (ES), cells of the rodent, or in fertilized rodent oocytes.

In a preferred embodiment, the deletion of variable domain diversity gene segments is conducted at three genomic loci encoding the immunoglobulin heavy, kappa, and lambda chains. Ideally, the deletion process is performed so that it leaves intact at least one of the *Adam6* genes that normally reside within the rodent heavy chain locus.

In a further preferred embodiment, the method of the invention produces an ES cell line homozygous for the three deletions that can be used for additional genomic engineering. Said genomic engineering may comprise a step, wherein a landing pad structure is inserted into all three deletion alleles. The landing pad being a genetic component that renders an engineered locus permissive for a recombinase-mediated cassette exchange (RMCE) procedure.

In a preferred embodiment, at least three ES cell lines are generated, one with a landing pad inserted into the locus that normally encodes the immunoglobulin heavy chain, one with a landing pad inserted into the locus that normally encodes the immunoglobulin kappa chain, and one with a landing pad inserted into the locus that normally encodes the immunoglobulin lambda chain. Preferably, the same kind of landing pad is inserted into each of these loci so that an identical form of RMCE can subsequently be used at all three genomic locations (i.e., a form of RMCE that depends on the same site-specific recombinase, or recombinases, in each case, and that involves the same arrangement of recombinase recognition sequences). Alternatively, different landing pads and thus different kinds of RMCE can be used in one or all cases.

Preferably, the landing pads are used for insertion of synthetic arrays of immunoglobulin variable domain gene segments whose coding sequences derive from humans or a species of veterinary significance.

In a further preferred embodiment, the engineering step that results in landing pad insertion also involves insertion of exons encoding constant domains in the required configurations. That is, at each of the light chain loci one or more constant domain exons are inserted so that the locus gains the capacity of expressing chimeric light chains comprising a rat light chain constant domain (either C_{Kappa} or C_{Lambda}). At the heavy chain locus, sequences are inserted to allow for:
(i) expression of rat IgM or IgD constant domains before class switch recombination;
(ii) expression of rat IgG2b or IgA constant domains after class switch recombination;
(iii) expression of rat IgG1 constant domains after Dre recombination.

Embryonic stem cells carrying engineered immunoglobulin loci that comprise this invention may be microinjected into a rodent blastocyst to create a chimeric rodent. Preferably, the cellular chimerism of the rodents produced extends to their germ cells allowing for the establishment - by breeding - of congenic mouse lines that are homozygous for one or all three of the engineered immunoglobulin loci.

### DESCRIPTION OF THE FIGURES

Figure 1 illustrates the deletion of variable domain gene segments from the mouse kappa chain locus and the insertion of new variable domain gene segments.
Figure 2 provides an overview of the heavy chain, kappa chain and lambda chain loci in the mouse genome.
Figure 3 exhibits the interval to be deleted (309) from the mouse immunoglobulin Kappa locus using CRISPR/cas9.
Figure 4 shows the interval to be deleted (408) from the mouse immunoglobulin Lambda locus using CRISPR/cas9.
Figure 5 exhibits the two intervals to be deleted (516 and 517) from the mouse locus that encodes the immunoglobulin heavy chain.
Figure 6 depicts a synthetic designed variable gene segment according to the present invention. The segment comprises a promoter (606), an exon containing untranslated sequence and the majority - if not all - of the coding sequence for the signal peptide (605), an intron (604), an exon containing the majority of the coding sequence for the immunoglobulin variable domain (603), a RAG-1/2 recombination signal sequence (602) and downstream (3') flanking DNA (601).

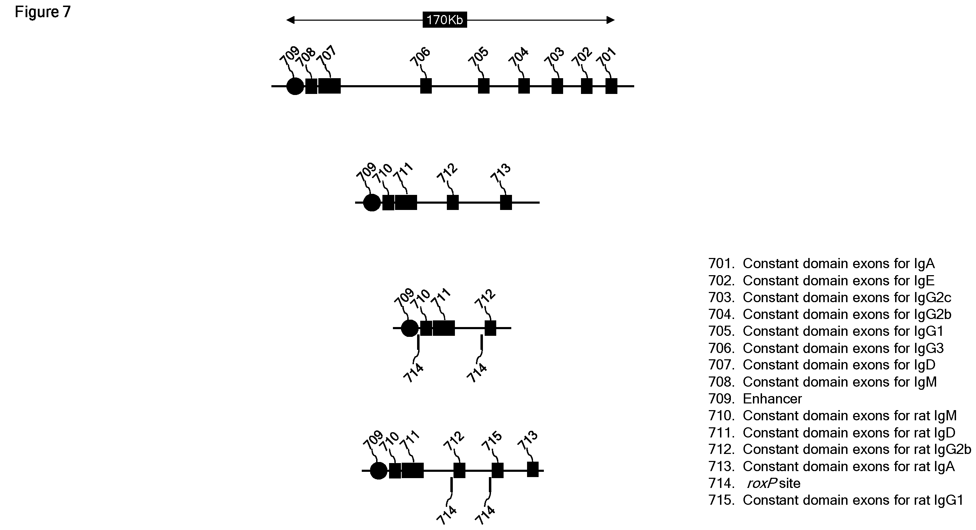

At top of Figure 7 is a depiction of the part of the mouse immunoglobulin heavy chain locus that contains the exons encoding constant domains. The second diagram from the top shows a minimal heavy chain constant domain locale of the present invention comprising the heavy chain enhancer (709) and the exons encoding the rat IgM (710), rat IgD (711), rat IgG2b (712), and IgA (713) constant domains. The third diagram from the top shows an alternative form of the constant domain locale of the present invention in which a *roxP* site (714) is found upstream of the rat IgM constant domain exons and also upstream of the rat IgG2b constant domain exons. The fourth diagram from the top shows an additional alternative form of the constant domain locale of the present invention in which *roxP* sites are found upstream of rat IgG2b (712) and rat IgG1 (715) constant domain exons. This last form of the constant domain locale would be permissive of AID-dependent isotype switching from IgM/IgD to IgG2b or IgA, and Dre recombinase-dependent switching from IgG2b to IgG1.

### DETAILED DESCRIPTION OF THE INVENTION

All publications, including but not limited to patents, patent applications and scientific publications, cited in this description are herein incorporated by reference for all purposes as if each individual publication were specifically and individually indicated to be incorporated by reference.

The use of the term "comprising" as well as other grammatical forms such as "comprises" and "comprised" is not limiting. The terms "comprising", "comprises" and "comprised" should be understood as referring to an open-ended description of an embodiment of the present invention that may, but does not have to, include additional technical features in addition to the explicitly stated technical features. In the same sense, the term "involving" as well as other respective grammatical forms such as "involves" and "involved" is not limiting. The same applies for the term "including" and other grammatical forms such as "includes" and "included". Section headings throughout the description are for organizational purposes only. In particular, they are not intended as limiting for various embodiments described therein, and it is to be understood that embodiments (and features therein) described under one subheading may be freely combined with embodiments (and features therein) described under another subheading. Further, the terms "comprising", "involving" and "including", and any grammatical forms thereof, are not to be interpreted to exclusively refer to embodiments that include additional features to those explicitly recited. These terms equally refer to embodiments that consist of only those features that are explicitly mentioned.

Provided in a first aspect of the present invention is a transgenic rodent, preferably a mouse, which is genetically engineered to be capable of expressing a functional repertoire of antibodies, preferably chimeric antibodies, with a reduced complement or repertoire of immunoglobulin heavy chain constant domains relative to the natural repertoire of immunoglobulin heavy chain constant domains found in common strains of laboratory rodents, e.g. laboratory mice. The transgenic rodent in all aspects of the invention is preferably a mouse.

The transgenic rodent, preferably a mouse, may be capable of producing one or more antibodies having IgM-, IgD-, IgG-, or IgA- immunoglobulin constant domains, said transgenic rodent, preferably a mouse, preferably capable of producing at least antibodies having at least IgM immunoglobulin constant domains.

Preferably, the transgenic rodent, preferably mouse is incapable of producing antibodies having IgE-constant domains.

In a further embodiment, in the transgenic rodent, preferably mouse, the variable domain gene segments that are normally present in an immunoglobulin chain locus have been deleted and replaced by one or more arrays of synthetic gene segments that are capable of undergoing V(D)J rearrangement to produce composite exons comprising open reading frames that encode one or more discrete protein domains. Preferably, the open reading frames encode one or more immunoglobulin variable domains, one or more immunoglobulin-related domains (i.e., domains from molecules that are not antibodies but which nonetheless adopt an immunoglobulin-related folding pattern - see Williams and Barclay, (1988) Ann. Rev. Immunol. 6:381-405)), or one or more domains with the potential for antigen-binding. The immunoglobulin-related domains that account for antigen binding by T cell receptors would be one preferred type of domain.

In a further embodiment, in the transgenic rodent, preferably mouse, the chimeric antibodies expressed by the transgenic rodent comprise one or more immunoglobulin constant domains with protein sequences taken from one or more species, each of which is not the rodent, preferably not from mouse. Preferably, one or more of the chimeric antibodies expressed by the transgenic rodent comprise immunoglobulin constant domains that are of rat origin in their amino acid sequences.

According to a preferred embodiment of the present invention, in the transgenic rodent, preferably mouse, the expressed chimeric antibodies may comprise one or more antibodies comprising IgM-, IgD-, or IgG- immunoglobulin constant domains. In a preferred embodiment, in the transgenic rodent, preferably mouse, the expressed chimeric antibodies further comprise antibodies comprising IgA- immunoglobulin constant domains.

In one embodiment, the transgenic rodent, preferably mouse, comprises an engineered immunoglobulin heavy chain locus capable of undergoing class switch recombination from IgM to IgG or IgA. In the engineered immunoglobulin heavy chain locus some or all forms of immunoglobulin class switching may be dependent on activation-induced cytidine deaminase (AID). Likewise, in the engineered immunoglobulin heavy chain locus some or all forms of immunoglobulin class switching may be dependent on one or more non-murine site-specific recombinases (such as Dre, Cre, Flp or other recombinases). Preferably, in the engineered immunoglobulin heavy chain locus, AID (activation-induced cytidine deaminase) regulates class switching from IgM to one isotype of IgG or to IgA, and a site-specific recombinase (such as Dre, Cre, Flp, or other recombinase) regulates switching from one isotype of IgG to a different IgG isotype.

In another preferred embodiment, in the transgenic rodent, preferably mouse, of the present invention, the expressed chimeric antibodies include chimeric light chains comprising rat immunoglobulin light chain constant domains.

The transgenic rodent, preferably mouse, of the present invention may comprise an engineered immunoglobulin light chain locus capable of expressing chimeric light chains that include a rat immunoglobulin light chain constant domain.

The transgenic rodent, preferably mouse of the present invention may comprise an engineered Kappa immunoglobulin light chain locus. The transgenic rodent, preferably mouse of the present invention may comprise an engineered Lambda immunoglobulin light chain locus.

In a further aspect of the present invention a cell of B-lymphocyte lineage (simply herein denominated "B cells") derived from the transgenic rodent, preferably mouse, of the present invention is provided.

In a further aspect of the present invention a method is provided for generating the transgenic rodent, said method comprising,
a) deleting variable domain gene segments from an immunoglobulin locus in the rodent genome,
b) inserting an array of synthetic gene segments into the modified immunoglobulin locus,
c) deleting one or more constant domain exons, from the same immunoglobulin locus in the rodent genome,
d) reinserting one or more constant domain exons, into the modified immunoglobulin locus, wherein the replacements comprise coding sequences of a different species from the original,
wherein steps a) to d) are preferably conducted in embryonic stem (ES), cells of the rodent, or in fertilized rodent oocytes; and wherein step c) may be performed with step a); and wherein the deletions and replacements may involve using contextual sequences such as those that regulate chromatin conformation, transcription, and heavy chain class switching.

In the method of the present invention, the engineering may be conducted at two genomic loci, one locus encoding the immunoglobulin heavy chain, and another locus encoding either the kappa or lambda immunoglobulin light chains.

In the method of the present invention, the engineering may be conducted at three genomic loci encoding the immunoglobulin heavy chain, and the loci encoding the kappa and lambda immunoglobulin light chains.

In the method of the present invention, preferably the deletion leaves intact at least one of the *Adam6* genes that normally reside within the rodent heavy chain locus.

In a preferred embodiment, in the method of the present invention, ES cell lines are produced that have deletions of variable domain gene segments from each of the three immunoglobulin loci, *i.e.,* the locus encoding immunoglobulin heavy chains and the two loci encoding immunoglobulin light chains. In one such embodiment, the ES cell lines carry only one form of deletion (*i.e.,* in one locus) in homozygous or heterozygous form. In other embodiments, the ES cell lines carry two or all three kinds of deletions (*i.e.,* in two or three loci, respectively) again in homozygous or heterozygous form, or a mixture of both forms.

In another preferred embodiment, in the method of the present invention, a landing pad structure is inserted into all three deletion alleles, rendering them permissive for recombinase-mediated cassette exchange (RMCE).

According to the present invention, at least three ES cell lines are preferably generated, one with a landing pad inserted into the locus that normally encodes the immunoglobulin heavy chain, one with the landing pad inserted into the locus that normally encodes the immunoglobulin kappa chain, and one with the landing pad inserted into the locus that normally encodes the immunoglobulin lambda chain.

In one embodiment, in the method of the present invention
a) one or more exons encoding a non-mouse immunoglobulin light chain constant domain are inserted in a downstream (i.e., 3') location to where variable domain gene segments are inserted, and
b) exons encoding non-mouse immunoglobulin heavy chain constant domains are inserted in a downstream location to where variable domain gene segments are inserted, and
c) the constant domain exons are inserted contemporaneously or prior to the insertion of the variable domain gene segments in each case, and
d) the constant domain exons encode polypeptides of rat origin.

According to the present invention, the embryonic stem cells may be microinjected into a rodent blastocyst to create a chimeric rodent exhibiting the features of the transgenic rodent according to the present invention.

A transgenic mouse platform for discovery of therapeutic antibodies of human or veterinary significance according to the present invention may be created in two general steps (as in **Figure 1****).** The first of these involves deletion of immunoglobulin variable domain diversity gene segments from the mouse genome using CRISPR/cas9 or related technology. The second step involves the reinsertion of new immunoglobulin variable domain diversity gene segments into the modified genome. A variation on the second step may involve the insertion of gene segments that can be recombined to form a non-antibody immunoglobulin domain, or a domain that does not adopt an immunoglobulin-related folding pattern. What follows immediately below are twelve embodiments describing components of the first step.

According to embodiment 1, the modifications may be conducted in mouse embryonic stem (ES) cells, but in some cases, they may also be conducted in fertilized mouse oocytes. If the former, mice are subsequently generated from the ES cells using standard blastocyst microinjection methodology. If the latter, mice derived from the fertilized oocytes may also be used to create ES cells to allow for additional genomic engineering to be performed.

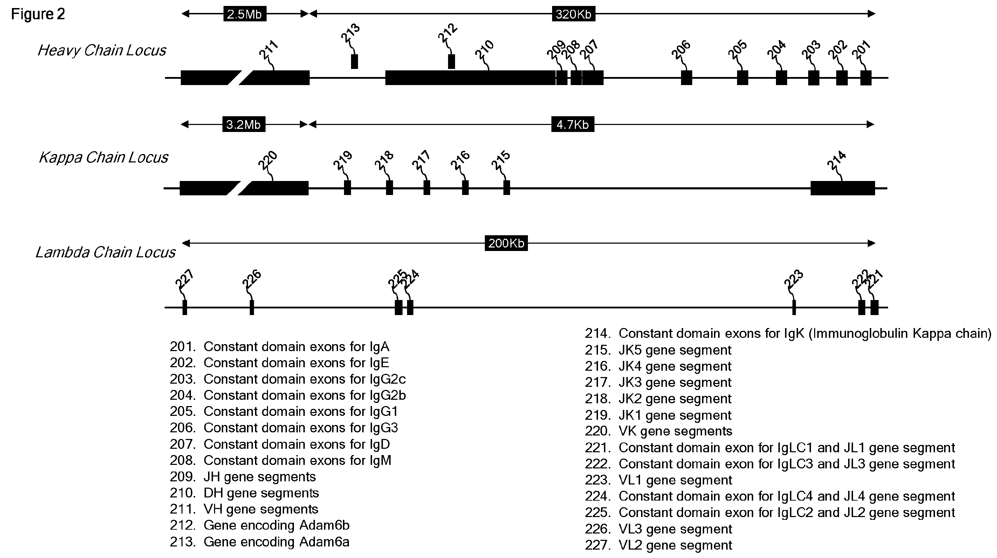

According to embodiment 2, the deletion of variable domain diversity gene segments is conducted at the three genomic loci encoding the immunoglobulin heavy, Kappa, and Lambda chains (**Figure 2**). These deletions may be accomplished by cas9-dependent homology-directed repair using DNA templates that share approximately five hundred base pairs of sequence identity on either side of the desired deletion junction. For the technology see e.g., Vanoli, F. et al., Proc Natl Acad Sci U S A 114, 3696-3701, 2017. The deletions may be engineered sequentially and/or simultaneously. Preferred is an ES cell clone that has biallelic deletions at all three immunoglobulin loci, that is, the cell line is homozygous for deletions of variable domain gene segments at the three loci.

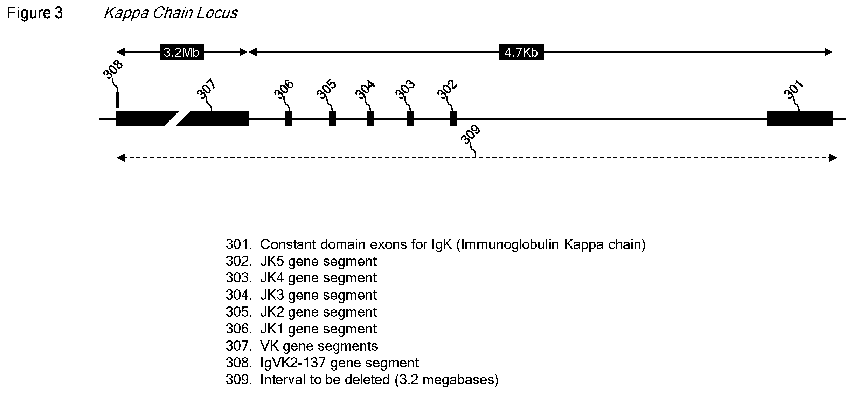

According to embodiment 3, the deletion at the mouse Ig-Kappa locus is approximately 3.2 megabases in size (**Figure 3**). The deleted interval may begin on the centromeric side of the gene segment for IgKV2-137 and may end on the telomeric side of IgCK (i.e., the gene segment that encodes the constant domain of the Kappa immunoglobulin light chain).

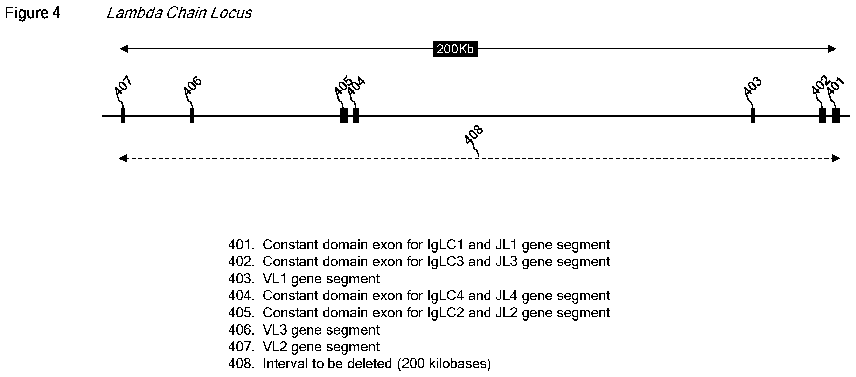

According to embodiment 4, the deletion at the mouse Ig-Lambda locus is approximately 200 kilobases in size (**Figure 4**). The deleted interval may begin on the telomeric side of IgVL2 and may end on the centromeric side of IgCL1.

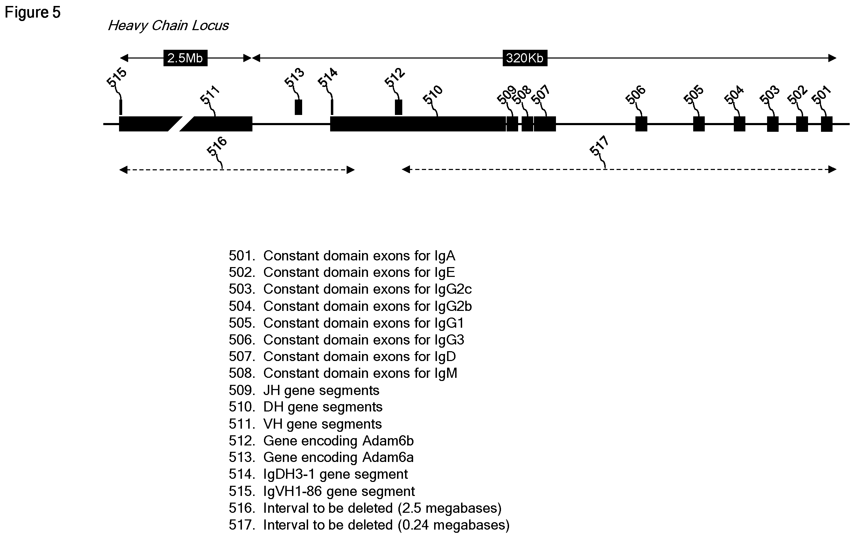

According to embodiment 5, the deletion at the mouse heavy chain locus is approximately 2.5 megabases in size (**Figure 5**). The deleted interval may begin on the telomeric side of IgVH1-86 and may end on the centromeric side of IgDH3-1. This deletion leaves intact one of the two Adam6 genes (*Adam6b*) that normally reside within the mouse heavy chain locus. It also leaves intact the D and J gene segments and all the constant domain exons located on the centromeric side of *Adam6b.* As explained below, a second engineering step at this locus may remove these remaining immunoglobulin gene segments and exons. An alternative deletion interval would extend from the telomeric side of IgVH1-86 and end on the telomeric side of the gene encoding Adam6a. This latter form of deletion may be preferred if it becomes important to retain both *Adam6* paralogs in a functional form; in this case, however, an additional engineering step (involving CRISPR/cas9-dependent homology-directed repair) is required to inactivate the two D gene segments that reside between *Adam6a* and *Adam6b.* Homozygous inactivation of both *Adam6* genes results in sterile male mice (see Voronina, V. et al., Biol Reprod. 100: 686-696, 2019) hence the value in retaining the genes in a functional unmodified form.

According to embodiment 6, the three deletions mentioned above create null alleles at the three immunoglobulin loci. That is, the modified alleles are incapable of expressing functional antibody chains. An ES cell line homozygous for the three deletions may be used to create mice carrying the three deletions. Interbreeding of such mice can create mice that lack the capacity to generate B cell antigen receptors and antibodies, and thus may feature abortive B lymphocyte development and a complete lack of mature B cells. Such mice are of benefit for accelerating downstream breeding programs as explained below.

According to embodiment 7, the three deletion alleles have a simple structure comprising an insertion that includes a drug resistance gene and/or a gene encoding a fluorescent protein. The former may allow for drug selection strategies designed to enrich for cells with monoallelic or biallelic deletions. The latter may also be used for a related purpose. The drug selection genes used preferably include those that confer resistance to neomycin, hygromycin, or blasticidin S, but other drug resistance genes may also be used instead. The fluorescent proteins used preferably include variants that fluoresce blue, green, or red, but other fluorescent proteins may also be used instead. Attempts may also be made to generate the deletions without inserting either a drug resistance gene or a gene encoding a fluorescent protein; if such marker-free deletions can be generated with high efficiency they may be preferred.

According to embodiment 8, and in addition to their use in creating antibody-deficient mice, ES cells homozygous for the three deletion alleles are used for additional genomic engineering. The ultimate purpose of this further engineering is to restore functionality to the deletion alleles by the insertion of variable domain gene segment arrays comprising sequences taken from species of veterinary interest. The secondary engineering preferably involves the insertion of "landing pad" structures into the deletion alleles; such structures rendering them permissive for recombinase-mediated cassette exchange (RMCE; see Turan, S. et al., Gene 515, 1-27, 2013). RMCE is then used for insertion of the variable domain gene segment arrays.

According to embodiment 9, a common landing pad structure is used for all three deletion alleles so that the same form of RMCE can be used at each location. This structure is preferably inserted into the deletion alleles by cas9-dependent homology-directed exchange using DNA repair templates comprising two components: **a.** flanking recognition sequences for the Cre recombinase that are mutually incompatible (e.g., *IoxP* and *Iox2272,* or alternatives of a related nature); **b.** a drug resistance gene that allows both positive and negative selection (*e.g.*, a gene comprising the coding sequence for the puromycin N-acetyltransferase enzyme from *Streptomyces alboniger* fused to an N-terminally truncated coding sequence for the thymidine kinase protein of Herpes simplex virus type 1). Alternative drug resistance genes may also be employed; an expressible coding region for a fluorescent protein could also be included within the landing pad (depending on the kind of methodology to be used during screening of ES cells).

According to embodiment 10, for cas9-dependent homology-directed repair, the DNA template used for landing pad insertion is flanked on either side with genomic DNA that is identical to that found in the immediate vicinity of the engineered deletion in the immunoglobulin locus being targeted. For the two light chain loci, the flanking DNA used for this purpose may be identical to that used to create the deletions of variable gene segment DNA. Thus, in these cases, the landing pad may simply replace the component (comprising a drug resistance gene and a gene encoding a fluorescent protein) that was inserted into the loci when the deletions were created. For the heavy chain locus, the flanking DNA may be used to create an additional deletion in the locus extending from a point just centromeric of the gene encoding Adam6b to a point centromeric of the terminal exon encoding the Igα constant domain.

According to embodiment 11, ES cells subjected to the form of cas9-dependent homology-directed repair just described are screened for biallelic insertions of the landing pad. Monoallelic insertions may also be permissive of the downstream RMCE procedure, but biallelic insertions may be preferred for simplicity and efficiency reasons. Three cell lines are ideally generated: one with a landing pad inserted into the locus that normally encodes the immunoglobulin heavy chain, one with a landing pad inserted into the locus that normally encodes the immunoglobulin kappa chain, and one with a landing pad inserted into the locus that normally encodes the immunoglobulin lambda chain.

According to embodiment 12, where necessary Flp recombinase (or other site-specific recombinases, or CRISPR/cas9) is used to remove unwanted DNA (such as expression units encoding drug resistance or fluorescent proteins) from the alleles. Specifically, there may be a need to remove the component inserted into the heavy chain locus when the initial 2.5 Mb deletion was created. This removal is desirable because it eliminates a transcription unit from a location in the heavy chain locus that might allow for it to impact V(D)J recombination or expression of the immunoglobulin chain. Analogous units in the other immunoglobulin loci may not require removal in this manner because, preferably, they may be removed coincident with insertion of the landing pads. This may not be the case for the heavy chain locus wherein the landing pad insertion takes place at a different location from the 2.5Mb deletion scar.

The product of the above is a minimum of three ES cell lines. Each cell line may carry a landing pad insertion (in mono- or bi-allelic form) in one of the three immunoglobulin loci. The landing pad may be permissive of RMCE allowing for the insertion of gene segment arrays into the immunoglobulin loci such that they become capable of generating antibody chains comprising variable domains derived from other species. The species of interest may be those of veterinary interest, such as dogs, cats or cattle, but, in principle, any species, including human, could be used as the source of the variable domains, or non-antibody domains could also be used.

The genetic background of the cell lines may be one that is incapable of expressing any functional antibody chains, and mice with this same background genotype may, in an embodiment of the invention, also be created. The genetic background is likely to be useful in accelerating downstream experiments intended to test the properties and functionality of any RMCE-modified immunoglobulin allele in mice.

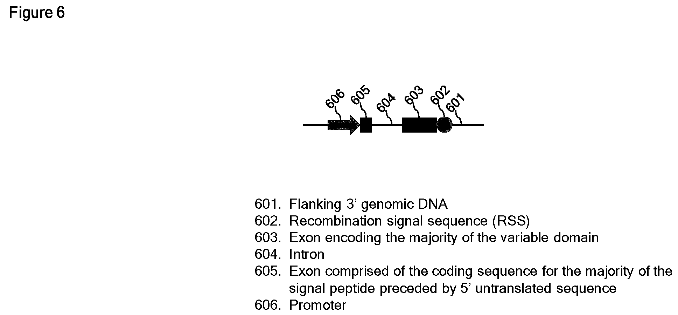

Gene segment arrays may be generated by DNA synthesis. A common design may be used for heavy and light chain V gene segments (**Figure 6**) involving the following components: minimal promoters (250-1000bp in length) from orthologous gene segments (these may be mined from the mouse, human, rat, or other species, with an emphasis on promoters from gene segments for which there is evidence of good incorporation in an expressed antibody repertoire); an exon comprising untranslated sequence and coding sequence for all or part of a signal peptide; an intron from an orthologous gene segment; an exon encoding the majority of a variable domain; a recombination signal sequence (RSS); flanking genomic DNA (mined from a region immediately downstream of an orthologous RSS).

In most, if not all, cases, different orthologous V gene segments may be used as the source of noncoding DNA for each synthetic V gene segment in an array. In most, if not all, cases, the same orthologous V gene segment may be used as the source of noncoding DNA for a single synthetic V gene segment. In some cases, synthetic V gene segments may use noncoding DNA from more than one source. The exons and intron may, however, have a common source, and if so this may mean that the noncoding intron may have a different source from the promoter. RSS sequences may also be derived from the source of the promoter, or the source of the exons, and/or they may have a non-natural origin (being designed instead from relevant consensus sequences).

In most, if not all, cases, promoters for use in arrays of heavy chain V gene segments may be taken from heavy chain V gene segments (from the mouse or orthologous immunoglobulin heavy chain loci). Similarly, in most, if not all cases, light chain V gene segment promoters (from the mouse or orthologous immunoglobulin light chain loci) may be used for the expression of light chain V gene segments in the arrays.

Genomes of species that have veterinary significance may be used as the source for the sequences found in the V gene segment exons present in the arrays. These may be mined from the available genome sequences either using existing annotation information or based on nucleotide and protein sequence homology.

Optimizations of various sorts may be made to the V gene segments. These may include changes to coding sequences such that preferred codons are used. Where possible the sequence may be modified to include sequences enriched for recognition by activation-induced cytidine deaminase, particularly in the DNA sequences that encode complementarity determining regions. Intron splice donor and acceptor sequences may be optimized where necessary. Recombination signal sequences may similarly be optimized - or adjusted to match a relevant consensus sequence - where necessary. Signal peptide cleavage sites may also be optimized as needed. Modifications may also be included that improve the likely readiness of the antibodies to be developed as therapeutics. Translation initiation sequences *(i.e.,* Kozak sequences) may be modified to increase conformity to the optimal consensus sequence. Cryptic translation initiation sequences and/or cryptic RSS sites in the V sequences, and/or splice donor or acceptor sequences, and/or cryptic recombination signal sequences may be removed where these are present.

J and D gene segments may be included in the arrays as appropriate (*i.e.,* both J and D gene segments may be used in the heavy chain arrays, while only J gene segments may be used in the light chain arrays). The coding sequences for these gene segments may be taken from the same genome that is used as the source for the V gene exons. The J and D gene segments may be embedded in a genomic context taken from one or more orthologous immunoglobulin loci (*i.e.,* a heavy chain J gene segment context may be used for J gene segments in the heavy chain array; a heavy chain D gene segment context may be used for D gene segments in the heavy chain array; and a light chain J gene segment context may be used for J gene segments in the light chain arrays). A minimum amount of noncoding (intergenic) DNA may be used between D gene segments in the arrays, and similarly a minimum amount of noncoding (intergenic) DNA may be used between J gene segments in the arrays.

Recombination signal sequences (RSSs) for use with array D and J gene segments may have an orthologous origin. This origin may be mouse, human or rat. Other species may be used if these sources do not prove sufficient. Where possible, RSSs equivalent to those found in the mouse immunoglobulin loci may be used *(i.e.,* RSSs with 12 base pair and 23 base pair spacers may be situated in the arrays in a manner that matches the arrangement found in the endogenous mouse immunoglobulin loci).

The arrays may be comprised such that the number of functional V, D and J gene segments they contain approximates that found in the relevant species of veterinary interest. If this species has a small number of a particular kind of gene segment (e.g., cattle are possessed of a relatively small number of heavy chain variable gene segments) duplicate variable gene segments may be included in the arrays with or without modest sequence adjustments as needed.

Pax5-activated intergenic repeat (PAIR) elements may be included in the arrays. Similarly, binding sequences for CCCTC-binding factor (*i.e.,* CTCF) may also be included in the arrays. The inclusion of these two kinds of elements may be made in a way that partially mimics their natural arrangement in immunoglobulin loci.

Constant domain exons may be included in the light chain arrays on the 3' side of the J gene segment arrays. Rats may be used as the preferred source of these exons. Transcriptional enhancer sequences (from the orthologous mouse or rat loci) may be added to the arrays to replace any that were deleted from the loci. The arrangement of constant domain exons and enhancer sequences may replicate that which is normally found in the light chain loci of rats, mice or humans. The mouse lambda chain locus contains two intergenic enhancer sequences, both of which may be included in the array that is constructed for insertion into the lambda chain locus.

The light chain arrays may be constructed such that after V(D)J rearrangement they allow for expression of chimeric antibody light chains comprising V domains whose protein sequence origin is human or a species of veterinary interest, and rat constant domains (or other non-mouse constant domains).

The heavy chain arrays may also contain rat constant domain exons. A minimum of three kinds of antibody constant domains may be included in the arrays, namely IgM, IgD and an IgG constant domain (e.g., the IgG2b constant domain; rat IgG2b is orthologous to mouse IgG2a and IgG2b; **Figure 7**). The array may include switch sequences (upstream of the IgM and IgG constant domain exons) so that class switching can occur from IgM to IgG during germinal center reactions. The heavy chain array may also include the transcriptional enhancer sequence that is normally located upstream of the IgM switch region. Exons encoding the IgA constant domain may also be included in the array because absence of IgA is associated with abnormalities in mice (and humans) that may impact antibody responses.

Activation-induced cytidine deaminase (AID) is required for class switch recombination (CSR) at the immunoglobulin heavy chain locus. The switch regions that participate in AID-dependent CSR are located upstream of the constant domain exons (with the exception of the IgD exons). These range in size (from 2-12Kb in the mouse) and are each composed of multiple tandem repeat sequences. The repetitive nature of the switch region DNA is likely to render it unstable during cloning in bacteria, and it may therefore prove challenging to generate synthetic arrays with these sequences appended. To avoid this kind of complication the rat constant domain exons may be inserted downstream of the heavy chain landing pad during the engineering step that may be used to delete the majority of the D and J gene segments (and the mouse constant domain exons) from the heavy chain locus. The alternative form of this step would create a deletion extending from the centromeric side of the *Adam6b* gene to the centromeric side of the IgA constant domain exon set. The DNA template used to repair this deletion would include a landing pad adjacent to an engineered piece of rat genomic DNA (cloned or synthesized DNA, or a combination of both). The rat DNA would include a minimal content of constant domain exons and associated switch regions as outlined above. In one version of the invention, the full complement of rat constant domain exons would be used. In an alternative version, deletions would be engineered into this full complement to derive a minimal set of the exons. The intronic enhancer sequence (normally located in the immediate vicinity of the IgM switch region) would be of mouse or rat origin. To improve the likelihood of isolating ES cells with the desired deletion and replacement, an additional positive selection gene may be included at the non-landing pad end of the template DNA. As above, to allow for CRISPR/cas9-mediated deletion and insertion, the template DNA would be flanked with sequences (of 300-1000bp in length) that are identical to the sequences flanking the guide RNA target sites. An alternative method for inserting the rat genomic DNA (with associated landing pad) could involve a form of RMCE that would not prohibit the form of RMCE to be used subsequently for insertion of DNA into the landing pad.

One possible form of the invention would include a switch from one heavy chain constant domain to another in a conditional manner. As an example, it may be useful to isolate monoclonal antibodies selectively from a cohort of antigen-specific B cells elicited at a specific stage in an immunization protocol. Methodology that could distinguish such cells from other antigen-specific B cells might enrich for antigen-binding characteristics that could be of particular interest. The conditional switch in constant domains could be accomplished, for example, by placing a recognition site for a sequence-specific recombinase such as the Dre recombinase upstream of the rat IgM constant domain set of exons (Dre recognizes *roxP* sites). A second *roxP* site would then be placed upstream of the exons encoding rat IgG2b (**Figure 7**). An S1pr2-Dre-ERT2 transgene (related to the transgene described in Shinnakasu, R. et al., Nat Immunol 17, 861-869, 2016) could then be used to induce selective elimination of the IgM and IgD exons in germinal center B cells in a conditional manner (by injecting mice with tamoxifen). The resultant cells would express antibodies using the rat IgG2b constant domain. Additional components could be included in this arrangement to enhance identification of the Dre-switched B cells and/or the antibodies they express. Alternatively, a related form of marking could be accomplished at the light chain loci (using epitope tags attached to the ends of the constant domains; see Schiepers, A. et al., Nature 615, 482-489, 2023).

V(D)J rearrangement of an immunoglobulin heavy chain locus carrying the above-described heavy chain variable domain gene segment array may result in expression of a chimeric antibody heavy chain. This chain may comprising a variable domain with a protein sequence sourced from humans or a species of veterinary interest (*e.g*., dogs, cats, or cattle) and a constant domain of rat origin (IgM, IgD, IgG2b, or IgA, and in one version of the invention - conditionally - IgG1).

Arrays of the sort just described may be inserted into the landing pads in the engineered immunoglobulin loci. The heavy chain array may be inserted into the heavy chain locus landing pad. The Kappa chain array may be inserted into the Kappa chain locus landing pad while the Lambda chain array may be inserted into the Lambda chain locus landing pad. Alternatively, the Kappa chain array may be inserted into both kinds of light chain landing pads and the same type of thing may be done with the Lambda chain array. These alternative insertions may provide opportunities for biasing light chain usage in different ways through the creation of alternative light chain genotypes in mice. Additional possibilities include the insertion of light chain variable domain arrays in the heavy chain locus and using only one of the two light chain loci (i.e., retaining homozygosity of a null allele at one of the two light chain loci).

For the insertions just mentioned, the arrays must be flanked by the same mutually incompatible Cre recombinase recognition sites used in the landing pads. If, as indicated above, one of these is a *IoxP* site, then Cre recombinase can catalyze recombination between a *IoxP* site at one end of a gene segment array and the landing pad *IoxP* site. In a similar fashion, Cre can catalyze recombination between a different recognition site at the other end of the gene segment array and the same site in the landing pad. The net result of this may be an exchange event in which the gene segment array is inserted into the landing pad between the mutually incompatible Cre recognition sites. Drug selection may be used to enrich for cells that have undergone the desired insertion event. On the one hand, if the landing pad contains the puro-TK fusion gene mentioned above, then ganciclovir may select against cells that retain this in their genome. On the other hand, if the gene segment array also contains a selectable marker (or more than one) then positive selection can be used for the desired insertion outcome.

If the cells used for RMCE have biallelic landing pad insertions, ganciclovir may select for cells that undergo biallelic RMCE events. Depending on the size of the gene segment array, such events may occur at a low frequency. With this in mind, therefore, it may be of benefit to generate cell lines that have monoallelic landing pad insertions, and to use these if difficulties arise at the RMCE stage.

Toxic HSV-TK-phosphorylated forms of ganciclovir can be transferred between cells through gap junctions (see Touraine, R. L. et al., Gene Ther 5, 1705-1711, 1998). This can lead to a significant bystander killing effect, which would be expected to reduce the apparent efficiency of RMCE (by the inappropriate killing of cells that have lost endogenous HSV-TK activity). This kind of effect could prove prohibitive if the desired RMCE outcome occurs infrequently. An alternative selection strategy based on complementation of an induced PigA (phosphatidylinositol glycan anchor biosynthesis class A) deficiency could be used to avoid this kind of problem (see Brosh, R. et al., Proc Natl Acad Sci U S A 118, 2021; see also Li, D. et al., Nucleic Acids Res 49, 2642-2654, 2021). In one preferred form, this would involve inactivating the *PigA* gene (located on the X-chromosome) with CRISPR/cas9 in ES cells prior to landing pad insertion. A landing pad would then be used that confers expression of PigA. PigA is required for the formation of glycosyl-phosphatidyl-inositol (GPI) anchors in cells, and in its absence, cells are devoid of proteins that use this type of plasma membrane attachment. Proaerolysin is a cytolytic pore-forming toxin from *Aeromonas hydrophila* that kills cells through a mechanism that depends on it binding to GPI anchors. It fails to kill cells that lack GPI anchors, such as those that lack PigA activity. Proaerolysin and PigA complementation have been used as the basis of an efficient counter selection strategy for genomic engineering. Optimally, such a strategy involves inactivating the endogenous *PigA* gene in a conditional manner such that native PigA expression can be restored to the ES cells prior to blastocyst injection.

The genomic engineering process outlined above involves use of multiple drug resistance genes. Some of them may be removed from the cells during landing pad insertion, in which case this may permit re-use of the removed drug resistance genes for positive selection during RMCE. Alternatively, the positive selection could be based on gain of a variant form of DHFR (carrying L22F and F31S mutations) which confers resistance to methotrexate (see Jonnalagadda, M. et al., Gene Ther 20, 853-860, 2013). One possible form of this selection strategy could involve placing the two components of a "split" form of the mutant DHFR at either end of a gene segment array. Alternative positive selection strategies may also be employed. Flp recombinase and/or another recombinase could subsequently be used for removal of any such drug resistance genes prior to creating mice with the ES cells.

At a minimum, the above process may yield three kinds of ES cells engineered for diversification of antibody variable domains with protein sequences derived from humans or a species of veterinary interest. One may be hemizygous or homozygous for an immunoglobulin heavy chain allele that has been reconstituted with a gene segment array. The other two may be hemizygous or homozygous for immunoglobulin light chain alleles (Kappa or Lambda). After deletion of drug resistance genes by site-specific recombination (or CRISPR/cas9 homology-dependent repair) these ES cells may be microinjected into mouse blastocysts to create chimeric mice using standard methodology, see e.g., Du, Y et al., Methods Mol Biol 1874, 99-114, 2019). Spectral karyotyping and other analytical procedures may be used to eliminate clones of cells that have common chromosomal abnormalities prior to microinjection. Southern blotting, polymerase chain reaction analysis, deep sequencing, and other analytical approaches may also be used to characterize the RMCE-generated alleles in detail before the clones are microinjected.

Chimeric mice may be bred to other mice to establish ES cell-derived lines of mice that carry the RMCE-generated alleles. Optimally, these other mice may be the ones created from ES cells that have large-scale deletions at all three immunoglobulin loci. Mice homozygous for these three deletions may lack expression of all antibody chains and thus may also lack B cells. ES cells carrying the RMCE-generated alleles may share this background genotype. F1 mice that are heterozygous for an RMCE-generated allele can be analyzed for expression of antibody chains from this kind of allele at early stages of B cell development. Similarly, F2 mice from an intercross of F1 heavy chain mice and F1 light chain mice can be analyzed for the presence of B cells and antibodies generated as a result of V(D)J rearrangement of the heavy and light chain alleles. Pilot antibody discovery campaigns can be performed using such early-stage mice using any standard immunization and antibody isolation methodology.

The functionality of the RMCE-generated alleles may be assessed using antibody discovery campaigns as just mentioned coupled with downstream analytical procedures that assess the antigen-binding properties of the derived antibodies. In parallel with such experiments, the mice may be interbred to generate stocks that are homozygous for desired combinations of heavy and light chain alleles. For instance, one preferred genotype may be homozygosity for RMCE-generated alleles at all three immunoglobulin chain loci. Alternative genotypes with biased light chain repertoires may also be created by the required form of interbreeding.

The mice created by the above process should be possessed of broad antibody chain repertoires. They should feature good antigen-specific repertoire diversity (due to usage of a large fraction of the available variable domain gene segments) to a range of antigens of different chemical natures. The antigen-specific antibody repertoires should show good evidence of somatic hypermutation as a consequence of germinal center reactions. Moreover, antigen-specific antibodies should include some that bind to their antigens with sub-nanomolar affinities. All of these properties are expected based on the design of the genomic engineering strategy and the known performance of other related immunoglobulin transgenic mouse platforms.

Key unique aspects of the current invention include the nature of the coding sequences of the gene segments present in the arrays, the promoters and noncoding sequences used to construct the arrays, the manner in which the gene segments and noncoding components are assembled in the arrays, the way in which Adam6 function is preserved in the heavy chain locus, the usage of rat constant domains in the creation of chimeric antibodies in the mice, the reduced complement of antibody isotype constant domains available at the heavy chain locus, and the engineering process for creating the alleles and mice. All these aspects are expected to contribute to platform performance.

In another aspect of the present invention, a method of producing antibodies or antigen-binding domains is provided comprising
a) immunizing the transgenic rodents of the present invention or
b) cultivating the B cells of the present invention.

In a preferred embodiment, the method includes a step of isolating the antibodies or antigen-binding domains expressed in the transgenic rodent of the present invention or in the B cells of the present invention. In this embodiment, e.g., conventional means can be employed such as bleeding the animals or removing blood, or lymphoid tissue or fluids from the animals. Other conventional means include the separation of the antibodies from the B cells. For example, upon culturing the B cells, the B cells may secrete antibodies into a medium or supernatant and the antibodies can be collected therefrom. The coding sequences of the antibodies can likewise be determined from genomic DNA and/or mRNA in the B cells.

In the present specification, the term "antigen-binding domain" is used to define the region of an antibody or antibody fragment that contains the complementarity-determining regions (CDRs) responsible for specific recognition and binding to an epitope on an antigen. An antigen-binding domain may also be derived from a T cell antigen receptor instead of an antibody. Furthermore, synthetic antigen-binding domains are also possible, these being synthetic moieties derived from protein domains that do not normally serve immunological antigen-binding functions, but which gain this property through engineering (adnectins, anticalins, DARPins and knottins are examples of this kind of synthetic antigen-binding domain).

The following Examples further describe the present invention. These examples are provided for purposes of illustration only, and are not intended to be limiting unless so specified. Thus, the present application should in no way be construed as being limited to the following examples, but rather, should be construed to encompass any and all variations which become evident as a result of the teaching provided herein.

### EXAMPLES

### Example 1: Deletion of variable domain gene segments from a mouse kappa chain locus and Insertion of new variable domain gene segments

An exemplary method illustrating the deletion of variable domain gene segments from a mouse kappa chain locus and insertion of new variable domain gene segments thereto is shown in **Figure 1****.** The genomic locus that encodes the mouse immunoglobulin Kappa chain is shown at the top of **Figure 1****.**

The variable gene segments (107) are found within a 3.2Mb region upstream of a region of about 5Kb containing the J gene segments (102-106) and the exon encoding the Kappa constant domain (101).

This locus can be reconfigured by a deletion and insertion process such that all the variability gene segments (V and J) are deleted and replaced with a "landing pad" structure (108) comprising mutually incompatible sequences recognized by a site-specific recombinase such as the Cre recombinase. New gene segments (109,110; synthetic with coding sequences sourced from a different genome) can be inserted en bloc, or in stages, into the landing pad by recombinase-mediate cassette exchange.

### Example 2: Overview of mouse heavy chain locus, kappa chain locus and lambda chain locus gene segments

**Figure 2** shows at top a depiction of the genomic locus that encodes the mouse immunoglobulin heavy chain.

The variable gene segments (211) are found within a 2.5Mb region upstream of a region of about 320Kb containing the D gene segments (210), the J gene segments (209) and the exons encoding the IgM (208), IgD (207), IgG3 (206), IgG1 (205), IgG2b (204), IgG2c (203), IgE (202) and IgA (201) constant domains. The locations of the genes encoding Adam6a (213) and Adam6b (212) are indicated.

The genomic locus that encodes the mouse immunoglobulin Kappa chain is depicted in the middle of **Figure 2****.**

The Kappa variable gene segments (220) are found within a 3.2Mb region upstream of a region of about 5Kb containing the J gene segments (215-219) and the exon encoding the Kappa constant domain (214).

The genomic locus that encodes the mouse immunoglobulin Lambda chain is depicted at the bottom of figure 2 (extending over approximately 200Kb).

Two V Lambda gene segments (V Lambda 2 [227] and V Lambda 3 [226]) are located upstream of the J Lambda 2 gene segment and the exon encoding C Lambda (225) which is adjacent to the J Lambda 4 gene segment and the exon encoding C Lambda 4 (224). The V Lambda 1 gene segment (223) lies further downstream, and this is upstream of the J Lambda 3 gene segment and the exon encoding C Lambda 3 (222); it is also upstream of the J Lambda 1 gene segment and the exon encoding C Lambda 1 (221).

### Example 3: Deletion of an interval responsible for variable domain diversification from the mouse immunoglobulin Kappa locus

**Figure 3** exhibits the interval to be deleted (309) from the mouse immunoglobulin Kappa locus using CRISPR/cas9.

The variable gene segments (307) are found within a 3.2Mb region upstream of a region of about 5Kb containing the J gene segments (302-306) and the exon encoding the Kappa constant domain (301). The location of the IgVK2-137 gene segment is indicated (308).

### Example 4: Deletion of an interval responsible for variable domain diversification from the mouse immunoglobulin Lambda locus

**Figure 4** shows the interval to be deleted (408) from the mouse immunoglobulin Lambda locus using CRISPR/cas9.

Two V Lambda gene segments (V Lambda 2 [407] and V Lambda 3 [406]) are located upstream of the J Lambda 2 gene segment and the exon encoding C Lambda (405) which is adjacent to the J Lambda 4 gene segment and the exon encoding C Lambda 4 (404). The V Lambda 1 gene segment (403) lies further downstream, and this is upstream of the J Lambda 3 gene segment and the exon encoding C Lambda 3 (402); it is also upstream of the J Lambda 1 gene segment and the exon encoding C Lambda 1 (401).

### Example 5: Deletion of an interval responsible for variable domain diversification from the mouse immunoglobulin heavy chain locus

**Figure 5** exhibits the interval to be deleted (516) from the mouse locus that encodes the immunoglobulin heavy chain.

The variable gene segments (511) are found within a 2.5Mb region upstream of a region of about 320Kb containing the D gene segments (510), the J gene segments (509) and the exons encoding the IgM (508), IgD (507), IgG3 (506), IgG1 (505), IgG2b (504), IgG2c (503), IgE (502) and IgA (501) constant domains. The locations of the genes encoding Adam6a (513) and Adam6b (512) are indicated as are the locations of the IgDH3-1 (514) and IgVH1-86 (515) gene segments.

### Example 6: A synthetic immunoglobulin variable gene segment

**Figure** 6 depicts a synthetic designed immunoglobulin variable gene segment.

The segment comprises a promoter (606), an exon containing untranslated sequence and the majority - if not all - of the coding sequence for the signal peptide (605), an intron (604), an exon containing the majority of the coding sequence for the immunoglobulin variable domain (603), a RAG-1/2 recombination signal sequence (602) and downstream (3') flanking DNA (601).

### Example 7: The immunoglobulin heavy chain locus that contains the exons encoding the constant domains

At top of **Figure 7** is a depiction of the part of the immunoglobulin heavy chain locus that contains the exons encoding the constant domains.

The figure shows the locations of the heavy chain enhancer (709) and the exons encoding the IgM (708), IgD (707), IgG3 (706), IgG1 (705), IgG2b (704), IgG2c (703), IgE (702) and IgA (701) constant domains. The second diagram from the top of the figure shows a minimal heavy chain constant domain locale comprising the heavy chain enhancer (709) and the exons encoding the rat IgM (710), rat IgD (711), rat IgG2b (712), and IgA (713) constant domains. The third diagram from the top of the figure shows an alternative version of the constant domain locale of the present invention in which a *roxP* site (714) is found upstream of the rat IgM constant domain exons and also upstream of the rat IgG2b constant domain exons. The fourth diagram from the top of the figure shows an additional alternative version of the constant domain locale of the present invention in which *roxP* sites are found upstream of rat IgG2b (712) and rat IgG1 (715) constant domain exons. This last form of the constant domain locale would be permissive of AID-dependent isotype switching from IgM/IgD to IgG2b or IgA, and Dre recombinase-dependent switching from IgG2b to IgG1.

## Claims

1. A transgenic rodent, which is genetically engineered to be capable of expressing a functional repertoire of antibodies, with a reduced complement of immunoglobulin heavy chain constant domains relative to the rodent that is not genetically engineered..

2. The transgenic rodent according to claim 1, wherein the rodent is a mouse.

3. The transgenic rodent according to claim 1 or 2, wherein the antibodies are chimeric antibodies.

4. The transgenic rodent according to any of claims 1 to 3, which is capable of producing one or more of antibodies having IgM-, IgD-, IgG-, or IgA- immunoglobulin constant domains and which is incapable of producing antibodies having IgE-constant domains.

5. The transgenic rodent according to any of claims 1 to 4, in which the variable domain gene segments that are normally present in an immunoglobulin chain locus have been deleted and replaced by one or more arrays of synthetic gene segments that are capable of undergoing V(D)J rearrangement to produce composite exons comprising open reading frames that encode one or more discrete protein domains.

6. The transgenic rodent according to claim 5, wherein the open reading frames encode one or more domains with the potential for antigen-binding, such as immunoglobulin variable domains or immunoglobulin-related domains, such as the domains found in T cell antigen receptors.

7. The transgenic rodent according to any of claims 3 to 6, wherein the chimeric antibodies expressed by the transgenic rodent comprise one or more immunoglobulin constant domains with protein sequences taken from one or more species, each of which is not the rodent, not from mouse origin and/or not from rat origin.

8. The transgenic rodent according to any of claims 1 to 7, wherein the complement of immunoglobulin heavy chain constant domains is reduced by deleting one or more of their native constant domain-encoding exons.

9. The transgenic rodent according to any one of claims 1 to 8, wherein the expressed chimeric antibodies comprise one or more of antibodies having IgM-, IgD-, or IgG-immunoglobulin constant domains and further comprise antibodies having IgA-immunoglobulin constant domains.

10. The transgenic rodent according to any of claims 1 to 9, comprising an engineered immunoglobulin heavy chain locus capable of undergoing class switch recombination from IgM to IgG or from IgM to IgA.

11. A cell of the B-lymphocyte lineage derived from the transgenic rodent, according to any of claims 1 to 10.

12. A method for generating the transgenic rodent of any one of claims 1 to 10, said method comprising,
a) deleting variable domain gene segments from an immunoglobulin locus in the rodent genome,
b) inserting an array of synthetic gene segments into the modified immunoglobulin locus,
c) deleting one or more constant domain exons from an immunoglobulin locus in the rodent genome,
d) reinserting one or more constant domain exons into the modified immunoglobulin locus, wherein the replacements comprise coding sequences of a different species from the original,
wherein steps a) to d) are conducted in embryonic stem (ES) cells of the rodent, or in fertilized rodent oocytes; and wherein deletion and reinsertion steps may be conducted sequentially or together; and wherein the deletions and replacements may involve using contextual sequences such as those that regulate chromatin conformation, transcription, and heavy chain class switching.

13. The method according to claim 12, wherein the engineering is conducted
a) at two genomic loci, one locus encoding the immunoglobulin heavy chain, and another locus encoding either the kappa or lambda immunoglobulin light chains, or
b) at three genomic loci encoding the immunoglobulin heavy chain, and the loci encoding the kappa and lambda immunoglobulin light chains.

14. The method according to claims 12-14, wherein the deletion leaves intact at least one of the *Adam6* genes that normally reside within the rodent heavy chain locus.

15. A method of producing and/or characterizing antibodies or antigen-binding domains comprising
c) immunizing the transgenic rodents of any of claims 1 to 10 or
d) cultivating the cells of claim 11.
